# EUROPEAN PATENT SPECIFICATION

(11) **EP 2 482 902 B1**
(45) Date of publication and mention of the grant of the patent: **05.08.2015**
(21) Application number: 10731563.2
(22) Date of filing: 14.06.2010
(51) Int. Cl.: A61M 15/00

(54) **INHALATOR FOR POWDER PREPARATIONS**
INHALATOR FÜR PULVERPRÄPARATE
INHALATEUR POUR PRÉPARATIONS PULVÉRULENTES

(30) Priority: 30.09.2009 IT MO20090240
(43) Date of publication of application: 08.08.2012
(73) Proprietor: OLIVA, Roberto, 41012 Carpi (Modena) (IT)
(72) Inventor: OLIVA, Roberto, 41012 Carpi (Modena) (IT)
(74) Representative: Brunacci, Marco
(86) International application number: PCT/IB2010/001432
(87) International publication number: WO 2011/039579

(56) References cited:
- WO-A1-2004/035121
- DE-A1- 4 004 904
- US-A- 5 201 308
- US-A- 5 699 789
- US-A- 5 964 417
- US-A1- 2007 277 821

## Description

### Technical Field

The present invention relates to an upgraded inhalator for powder preparations.

### Background Art

Inhalators for powder preparations are known requiring the use of capsules each containing a dose of preparation. The capsules are individually inserted inside the inhalator which, in different ways, allows breaking the capsule and making the corresponding dose of powder preparation available for inhalation.

The inhalators of this type are generally composed of a container comprising an inhalation channel put in communication with a housing chamber for housing the capsule containing the medicinal product to be inhaled.

Associated with the housing chamber are means for breaking the capsule, arranged in correspondence to the extremities of the housing chamber respectively and substantially composed of two perforators having, respectively, contrast springs and a certain number of aculei which are able to penetrate, by applying a certain pressure with the fingers, inside the housing chamber itself. The powder can this way come out of the capsule and be inhaled by the user. The inhalators of this type however have numerous drawbacks, tied mainly to the high number of component parts, such as the aculei, the springs and the extremities required to operate the perforators. Some of such components are in relative motion with respect to the container and malfunctions and blockages are therefore possible.

Furthermore, the multiple use involves the bacteriological contamination of the inhalator by means of the saliva or external agents such as dust, and consequently cleaning operations can become necessary.

Again, these known inhalators require accurate assembly operations to be able to operate correctly, and these involve a consequent increase in production costs and therefore make single-use economically inopportune.

Part of these drawbacks have been overcome by an inhalator presented in the international patent application WO 2004/035121 made by the same applicant and which envisages the presence of a main body, having an inhalation channel, to which is secured in rotation a secondary body having a housing in which are inserted the capsules containing the medicinal product to be inhaled.

The secondary body can turn between an opening position, wherein the housing is accessible from outside for inserting the capsules, and a closing position, wherein the housing is in communication with the inhalation channel. This type of inhalator also has cutting means suitable for cutting a portion of the capsule protruding from the housing during the rotation of the secondary body from the opening position towards the closing position, so the contents of the capsule run into the inhalation duct.

This type of inhalator, however more functional and reliable with respect to the inhalators initially described, is not adequate for the use of a loose powder preparation, i.e, not enclosed in a capsule, to be released at the time of use and also has the above drawbacks relating to contamination by saliva and dust.

For this reason, the present applicant has provided a further inhalator for powder preparations, described in the patent application EP 1 725 287.

EP 1 725 287 describes a disposable and single-dose inhalator, composed of a first body, having an inhalation channel, and a second body, having a tank for containing a dose of a powder preparation to be inhaled, where the first body is moving in rotation with respect to the second body to allow the powder preparation to fall from the tank into the inside of the inhalation channel.

The rotation of the first body with respect to the second body therefore allows putting in communication or selectively isolating the tank from the inhalation channel.

As any technician in the sector knows, the quantities of active medicinal product for lung inhalation are so small, i.e., less than one milligram per dose, that the sharing out into single doses can be problematic; for this reason, in most cases, the loose powder preparation to be inhaled also comprises a carrying component (also called "carrier"), composed of one or more inert substances made up of particles of larger dimensions compared to those of the active ingredient, which allows the preparation of single doses of active medicinal product in a reproducible way.

However, to ensure that inhalation is efficient, i.e., that the active ingredient is actually inhaled at lung level (i.e., that it reaches the peripheral part of the lung) by the user, during transit through the inhalation channel, the particles containing the active ingredient must separate from the carrying particles.

For this reason, the inhalator described in EP 1 725 287 has protuberances along the inhalation channel, designed to be impacted with the powder preparation during the crossing of the inhalation channel itself towards the outside, i.e., towards the user.

Such protuberances, which therefore have the function of separating the particles containing the active ingredient from the carrying particles, have a sloped surface turned towards the external opening of the inhalation channel accessible by the user.

This type of inhalator for powder preparations also has a number of drawbacks. In fact, laboratory tests made on this type of inhalator show that the quantity of the particles of active ingredient freed in the impact with the above-mentioned protuberances and suitable for being absorbed at peripheral lung level (called "breathable fraction") is lower than that of the powder inhalator devices available on the market.

The quantity of particles freed by the impact with the protuberances in the inhalator described in EP 1 725 287 is not therefore large enough to consider inhalation effective and satisfactory.

Another drawback of the inhalator described in EP 1 725 287 is related to tank loading which is not at all easy and functional.

In fact, as explicitly described in EP 1 725 287, to load the tank, the first body has to be removed from the second body so as to make the tank accessible from the outside. As it is easy to appreciate, this operation is not at all practical as it requires the disassembly of the inhalator itself.

DE 40 04 904 discloses a pocket drum applicator for multiple inhalation of powdery drugs.

The applicator disclosed by DE 40 04 904 comprises an exchangeable reservoir sealed against the ambient atmosphere and suitable to display the remaining quantity. The drug is withdrawn by means of a rotatable drum with modifiable dose volume and active, adjustable expulsion of the powdery drug. The drug is atomized in a separating chamber with a flow pressure adapted to the size of the particles. To operate the drum applicator, a button need be pushed once only. Patent document WO2004/035121 A1 discloses an inhalator which comprises a main body defining an inhalation conduit, and a second body exhibiting a housing, into which a capsule can be at least partially inserted, wherein the capsule is provided for being cut off by moving the second body on the main body. However this inhalator is limited to be refilled with a powder filled capsule and is not suitable to be refilled with a powder preparation in loose form.

### Description of the Invention

The main object of the present invention is to provide an upgraded inhalator for powder preparations that allows obtaining a breathable fraction of active medicinal product greater than that obtained with the inhalator described in EP 1 725 287.

The present invention therefore wishes to provide an inhalator for powder preparations having greater efficiency, i.e., that allows performing more effective inhalation compared to known inhalators and in particular compared to the inhalator described in EP 1 725 287.

One object of the present invention is to provide an inhalator for powder preparations that allows the easier and more functional loading of the tank designed to contain the powder preparation compared to the inhalators of known type.

Another object of the present invention is to provide an upgraded inhalator for powder preparations which allows overcoming the mentioned drawbacks of the state of the art within the ambit of a simple, rational, easy and effective to use as well as low cost solution.

The above objects are all achieved by the present inhalator for powder preparations, according to the invention, that has the features set forth in claim 1.

### Brief Description of the Drawings

Other characteristics and advantages of the present invention will become more evident from the description of a preferred, but not sole, embodiment of an upgraded inhalator for powder preparations, illustrated purely as an example but not limited to the annexed drawings in which:
figure 1 is a perspective view of the inhalator according to the invention in a preferred embodiment;
figure 2 is a longitudinal section view of the inhalator of figure 1 with the first body in the first work position;
figure 3 is a longitudinal section view of the inhalator of figure 1 with the first body in the second work position;
figure 4 is a perspective view of the exploded view of the inhalator of figure 1; figure 5 is a perspective view of the inhalator according to the invention in an alternative embodiment;
figure 6 is a longitudinal section view of the inhalator of figure 5 with the first body in the first work position;
figure 7 is a longitudinal section view of the inhalator of figure 5 with the first body in the second work position.

### Embodiments of the Invention

With reference to such figures, globally indicated by 1 is an upgraded inhalator for powder preparations.

The inhalator 1 comprises a main body 2 having at least an inhalation channel 3 having at least an external opening 4 accessible by a user, at least a tank 5 designed to contain at least a dose of powder preparation P and which can be opened to be put in communication with the channel 3 and at least a protuberance 6a, 6b protruding towards the inside of the channel 3.

As is known to experts in the sector, by powder preparation is meant a series of particles comprising the particles of the active ingredient to be inhaled and the carrying particles.

According to the invention, the protuberance 6a, 6b defines a substantially concave impact surface 7a, 7b arranged on the opposite side with respect to the external opening 4. The impact surface 7a, 7b is therefore destined to be intercepted by the powder preparation P during the crossing of the channel 3 towards the external opening 4.

Preferably, the impact surface 7a, 7b is curvilinear and is substantially C-shaped or hook-shaped.

The impact surface 7a, 7b is therefore shaped so as to define a tortuous path that must be crossed by the powder preparation P during its movement towards the external opening 4, so as to obtain a highly turbulent flow of air inside the channel 3 and facilitate the detachment of the particles containing the active ingredient from the carrying particles.

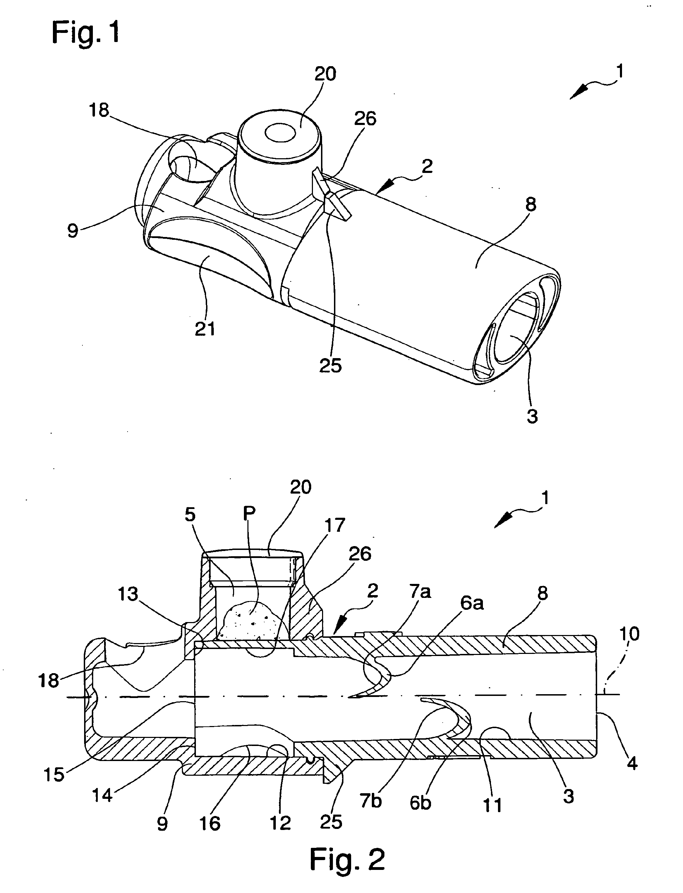

In a preferred embodiment, shown in figure 1, the inhalator 1 comprises two protuberances 6a and 6b, wherein a first protuberance 6a and a second protuberance 6b define a first impact surface 7a and a second impact surface 7b respectively.

The protuberances 6a and 6b are arranged spaced to each other along the channel 3 and are arranged on the opposite sides with respect to a median plane of the inhalation channel itself.

Advantageously, as shown in the embodiment shown in the illustrations, the main body 2 comprises a first body 8, wherein the inhalation channel 3 is defined, and a second body 9, wherein the tank 5 is defined.

Suitably, both the first body 8 and the second body 9 are made of transparent material, so that the user can see from outside the fall of the powder preparation inside the channel 3.

The first body 8 is mobile with respect to the second body 9 between at least a first position, wherein it closes the tank 5 isolating it from the channel 3, and at least a second position, wherein it opens the tank 5 putting it in communication with the channel 3.

Preferably, the first body 8 has an elongated shape and the channel 3 extends along the longitudinal axis of the first body itself, identified in the illustrations with reference number 10.

More in particular, in the embodiment shown in the illustrations, the first body 8 has a substantially oval outer section and a substantially round internal section defined by the side surface 11 of the channel 3.

The protuberances 6a and 6b protrude from the side surface 11 of the channel 3 crossways to the longitudinal axis 10, are spaced to each other along the longitudinal axis itself and are arranged on the opposite sides with respect to a longitudinal median plane of the channel 3.

Suitably, the second body 9 comprises a housing seat 12 for at least a portion of the first body 8, which is rotating mobile with respect to the second body 9 inside the housing seat itself.

In the embodiment shown in the illustrations, the first body 8 is rotating mobile with respect to the second body 9 around the longitudinal axis 10.

More in particular, the housing seat 12 has a mechanical stop 13 designed to be contacted by a counter-stop 14 defined by the portion of the first body 8 which can be fitted inside the housing seat itself.

The inhalator 1 then comprises rotation stop means suitable for indicating to the user that the end of stroke has been reached during the rotation of the first body 8 with respect to the second body 9.

These stop means are e.g. made up of a relief and of a recess defining a stop and a counter-stop respectively suitable for cooperating when the first body 8 reaches the above-described work positions. More in particular, the stop means are shaped so as to also produce a sound signal when the first body 8 reaches the end-of-stroke position.

Advantageously, the channel 3 is of the through type and has a first port 15, arranged on the opposite side to the external opening 4 with respect to the protuberances 6a and 6b, and a second port 16, defined on the side surface 11 and suitable for putting in communication the tank 5 with the channel 3 in the second position of the above-described first body 8. Suitably, the first and the second port 15 and 16 are defined in the portion of the first body 8 insertable in the housing seat 5 and the second port 16 is placed between the external opening 4 and the first port 15.

In the preferred embodiment shown in the illustrations, the external opening 4 and the first port 15 are defined in correspondence to the axial extremities of the first body 8.

More in particular, the axial extremity of the first body 8 defining the first port 15 also defines the counter-stop 14 designed to rest on the mechanical stop 13 of the housing seat 12.

Preferably, the second port 16 is put in communication with the first port 15 thereby defining a single port extending between an axial extremity and a portion of the external side surface of the first body 8.

The side surface 11 also has a recess 17 defined in correspondence to an area substantially longitudinally aligned with the first protuberance 6a and substantially facing the second port 16. The area of the side surface 11 facing the second port 16 is therefore lowered with respect to the surrounding areas of the side surface itself.

The impact surfaces 7a and 7b of the protuberances 6a and 6b are suitably turned towards the first port 15 and the first protuberance 6a is arranged upstream of the second protuberance 6b with respect to the crossing direction of the channel 3 by the powder preparation P, direction which is oriented from the inside of the channel itself towards the external opening 4.

Moreover, the first protuberance 6a is arranged on the opposite side to the second protuberance 6b and to the second port 16 with respect to the median plane of the channel 3.

The second body 9 then defines an air inlet mouth 18 communicating with the inhalation channel 3.

More in particular, the inlet mouth 18 is put in communication with the bottom wall of the housing seat 12 which defines the stop 13 and is therefore put in communication with the first port 15 in turn arranged in correspondence to the axial extremity of the first body 8 defining the counter-stop 14.

In the preferred embodiment of the inhalator 1, shown in the figures from 1 to 4, the tank 5 has an insertion mouth 19 for the powder preparation P accessible from the outside and directly communicating with the tank itself. The insertion mouth 19 is therefore defined on the second body 9 in such a way that the tank 5 is accessible from the outside, whatever the position of the first body 8.

The inhalator 1 then comprises a cover 20 associated in a removable way with the second body 9 and suitable for closing the insertion mouth 19.

Preferably, the inhalator 1 can be equipped with a plurality of covers 20 of different colour, each of which identifies a different type of medicinal product used.

In an alternative embodiment, shown in the figures from 5 to 7, the tank 5 is closed at the top, i.e., the second body 9 has a closing wall 23 defined all in one piece with the second body itself.

The second body 9 then comprises an opening 24 facing the tank 5 and arranged on the opposite side with respect to the closing wall 23. Such opening 24 can be usefully used to introduce the powder preparation inside the tank 5 before the first body 8 is inserted into the housing seat 12.

The opening 24 communicates therefore with the tank 5 when the first body 8 is removed from the housing seat 12, while it is isolated from the tank 5 when the first body 8 is inserted into the housing seat 12.

In both the embodiments shown, the second body 9 has two grip areas 21 on its outer surface that can be engaged by the user and having an ergonomic shape. The grip areas 21 thus identify the grip of the inhalator 1 in a univocal way. Advantageously, the inhalator 1 has identification means 25, 26 of the reciprocal position of the bodies 8 and 9 suitable for allowing blind users to easily determine the position of the first body 8 with respect to the second body 9.

More in detail, the identification means 25, 26 comprise a first fin 25 arranged on the outer surface of the first body 8 and a second fin 26 arranged on the outer surface of the second body 9 and arranged beside the tank 5.

The first fin 25 is therefore on the opposite side to the second fin 26 with the first body 8 in the first work position, while it is substantially aligned with the second fin 26 when the first body 8 is in the second work position.

The operation of the present invention is as follows.

After inserting the portion of the first body 8 comprising the ports 15 and 16 inside the housing seat 12, the first body itself is conveyed to the first work position, so that its outer side surface closes the tank 5 isolating it from the channel 3.

Subsequently the cover 20 is removed from the second body 9 so that the tank 5 is accessible from outside for the insertion of the powder preparation P.

Once the powder preparation P has been introduced inside the tank 5, the latter is closed with the cover 20.

This situation is shown in figure 2, where the tank 5 contains a dose of powder preparation P and the first body 8 is in the first work position.

In the alternative embodiment of the inhalator according to the invention, shown in the figures from 5 to 7, the powder preparation must be introduced inside the tank 5 through the opening 24 before inserting the first body 8 in the housing seat 12. The introduction of the first body 8 in the housing seat 12, positioned so the second port 16 is in correspondence to the opening 24, then allows maintaining the powder preparation inside the tank 5.

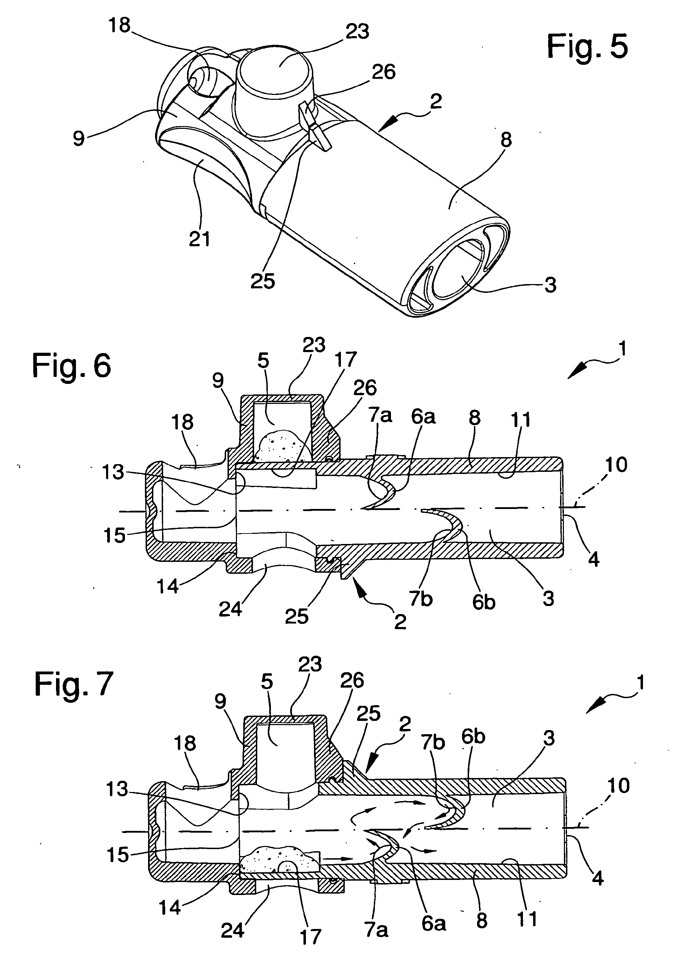

This situation is shown in figure 6, where the tank 5 contains a dose of powder preparation P and the first body 8 is in the first work position.

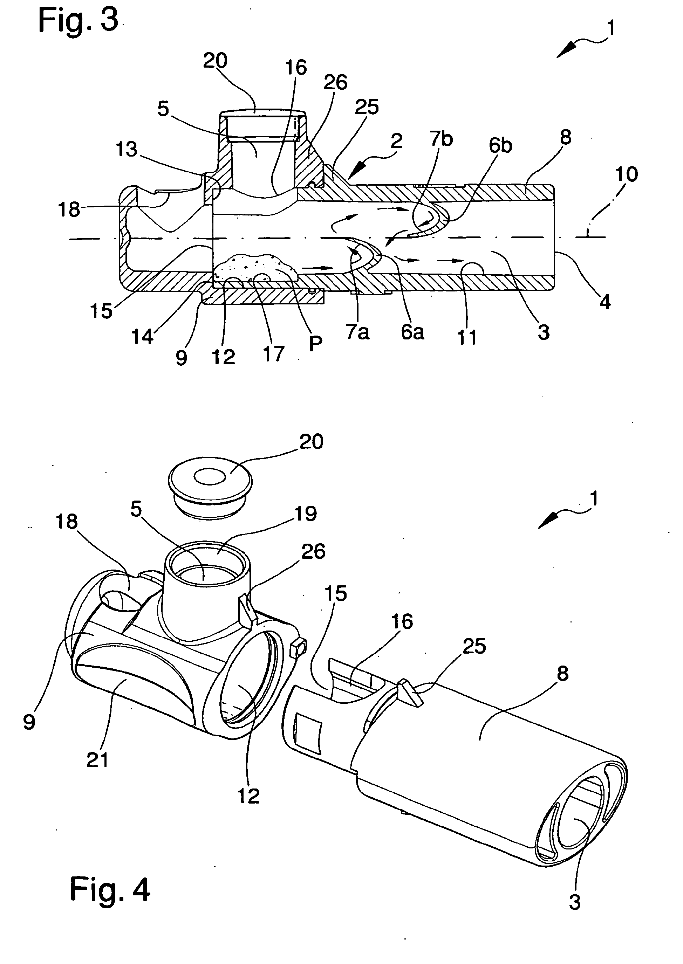

The user can therefore rotate the first body 8 with respect to the second body 9 by an angle substantially equal to 180°, bringing it to the second work position wherein the second port 16 is in correspondence to the tank 5. In this configuration, shown in the figures 3 and 7, the tank 5 is therefore put in communication with the channel 3 and the powder preparation P falls by gravity inside it, resting on the side surface 11. More precisely, when the tank 5 is opened, the powder preparation P is collected up in correspondence to the recess 17.

The fall of the powder preparation can be displayed by the user through the transparent walls of the first and of the second bodies 8 and 9.

At this point, the user introduces the external opening 4 inside his/her mouth and breathes in the air from the inlet mouth 18, thereby generating a flow of air inside the channel 3 directed towards the external opening 4.

This flow of air crossing the inhalation channel 3 reaches the powder preparation that has collected up inside the channel itself and drags this in its movement towards the external opening 4.

During the crossing of the channel 3 towards the external opening 4, the powder preparation P initially encounters the first impact surface 7a, which is substantially aligned longitudinally with the recess 17 on which the powder preparation itself collects up, and subsequently the second impact surface 7b, arranged downstream of the first protuberance itself with respect to the direction of movement of the powder preparation P inside the channel 3.

The C shape of the first and the second impact surfaces 7a and 7b gives the channel 3 a winding course such as to make the flow of air that conveys the powder preparation P towards the external opening 4 completes a substantially tortuous path identified by the arrows 22 shown in the figures 3 and 7.

More in particular, the concave shape of the impact surfaces 7a and 7b is intended to cause substantial turbulence inside the channel 3, so as to increase the number of knocks of the particles of powder preparation P against the surfaces defined by the protuberances 6a and 6b and against the side surface 11 of the inhalation channel itself, as well as between the particles themselves.

It has in point of fact been ascertained how the described invention achieves the proposed objects and in particular the fact is underscored that the inhalator according to the invention allows obtaining a more efficient inhalation with respect to the known inhalators, i.e., it substantially allows obtaining a high and effective breathable fraction.

More in particular, the concave shape characterising the impact surfaces defined by the protuberances of the inhalator results in the powder preparation having to complete a particularly tortuous path in its movement towards the mouth of the user and such as to cause a high number of knocks of the particles of the powder preparation against the walls delimiting the inhalation channel itself and between the particles themselves.

The inhalator according to the invention, furthermore, allows the easy filling of the tank, and is therefore considerably more functional and practical than the inhalators of known type available on the market.

## Claims

1. Inhalator (1) for powder preparations, comprising a main body (2) having:
- at least a first body (8) having an elongated shape and defining an inhalation channel (3) extending along a longitudinal axis (10) of said first body (8), with said inhalation channel (3) having at least an external opening (4) accessible by a user;
- at least a second body (9) defining a tank (5)suitable to contain at least a dose of powder preparation (P) and to be opened so as to be put in communication with said inhalation channel (3); and
- at least a protuberance (6a, 6b) protruding from the side surface (11) of said inhalation channel (3) crossways to said longitudinal axis (10);
wherein said first body (8) is mobile in rotation with respect to said second body (9) around said longitudinal axis (10) between at least a first position, in which said first body (8) closes said tank (5) isolating it from said inhalation channel (3), and at least a second position, in which said first body (8) opens said tank (5) putting it in communication with said inhalation channel (3), and
wherein said protuberance (6a, 6b) defines at least a substantially concave impact surface (7a, 7b) arranged on the opposite side to said external opening (4), said impact surface (7a, 7b) being therefore intended to be intercepted by said powder preparation (P) during the crossing of said inhalation channel (3) towards said external opening (4);
the inhalator (1) being **characterized by the fact** that said tank (5) has an insertion mouth (19), for inserting said powder preparation (P), accessible from outside, and that said impact surface (7a, 7b) is substantially C-shaped.

2. Inhalator (1) according to the claim 1, **characterised by** the fact that it comprises two of said protuberances, respectively a first and a second protuberance (6a, 6b), arranged spaced to each other along said inhalation channel (3) and arranged on opposite sides with respect to a median plane of the inhalation channel itself.

3. Inhalator (1) according to claim 1 or 2, **characterised by** the fact that said second body (9) comprises a housing seat (12) of at least a portion of said first body (8), the latter being mobile in rotation with respect to said second body (9) inside said housing seat (12).

4. Inhalator (1) according to one or more of the preceding claims, **characterised by** the fact that said inhalation channel (3) is of the through type and has a first port (15) arranged on the opposite side to said external opening (4) with respect to said at least one protuberance (6a, 6b) and at least a second port (16) defined on its side surface (11), said first and said second ports (15, 16) being defined in the portion of said first body (8) insertable in said housing seat (12) and said second port (16) being suitable for putting in communication said tank (5) with said inhalation channel (3) in said second position of the first body (8).

5. Inhalator (1) according to claim 4, **characterised by** the fact that said external opening (4) and said first port (15) are arranged in correspondence to the axial extremities of said first body (8) and by the fact that said second port (16) is placed in between said external opening (4) and said first port (15).

6. Inhalator (1) according to claim 4 or 5, **characterised by** the fact that said impact surface (7a, 7b) is turned towards said first port (15).

7. Inhalator (1) according to any preceding claim as depending on claim 2, **characterised by** the fact that said first protuberance (6a) is arranged upstream of said second protuberance (6b) and on the opposite side to said second port (16) with respect to said median plane.

8. Inhalator (1) according to any preceding claim, **characterised by** the fact that said second body (9) comprises an air inlet mouth (18) communicating with said inhalation channel (3).

9. Inhalator (1) according to any preceding claim, **characterised by** the fact that it comprises at least a cover (20) associated in a removable way with said second body (9) and suitable for closing said insertion mouth (19).

## Patentansprüche

1. Inhalator (1) für pulverförmige Zubereitungen, umfassend einen Grundkörper (2) mit:
- mindestens einem ersten Körper (8), der eine langgestreckte Form aufweist und einen Inhalationskanal (3) definiert, der sich entlang einer Längsachse (10) des ersten Körpers (8) erstreckt, wobei der Inhalationskanal (3) mindestens eine äußere Öffnung (4) aufweist, die durch einen Anwender zugänglich ist;
- mindestens einem zweiten Körper (9), der einen Behälter (5) definiert, welcher geeignet ist, um mindestens eine Dosis einer pulverförmigen Zubereitung (P) zu enthalten und um geöffnet zu werden, um mit dem Inhalationskanal (3) in Verbindung gebracht zu werden; und
- mindestens einer Protuberanz (6a, 6b), die von der Seitenfläche (11) des Inhalationskanals (3) quer zu der Längsachse (10) hervorsteht;
wobei der erste Körper (8) in Bezug auf den zweiten Körper (9) um die Längsachse (10) drehbeweglich ist zwischen mindestens einer ersten Position, in welcher der erste Körper (8) den Behälter (5) verschließt, wodurch dieser von dem Inhalationskanal (3) isoliert wird, und mindestens einer zweiten Position, in welcher der erste Körper (8) den Behälter (5) öffnet, wodurch dieser in Verbindung mit dem Inhalationskanal (3) gebracht wird, und
wobei die Protuberanz (6a, 6b) mindestens eine im Wesentlichen konkave Prallfläche (7a, 7b) definiert, die an der gegenüberliegenden Seite zu der äußeren Öffnung (4) angeordnet ist, wobei die Prallfläche (7a, 7b) dementsprechend dazu vorgesehen ist, um von der pulverförmigen Zubereitung (P) während der Durchquerung des Inhalationskanals (3) in Richtung zu der äußeren Öffnung (4) unterbrochen zu werden;
wobei der Inhalator (1) **dadurch gekennzeichnet ist, dass** der Behälter (5) eine Einführöffnung (19) zum Einführen der pulverförmigen Zubereitung (P) aufweist, die von außen zugänglich ist und dass die Prallfläche (7a, 7b) im Wesentlichen C-förmig ist.

2. Inhalator (1) nach Anspruch 1, **dadurch gekennzeichnet, dass** er zwei der Protuberanzen aufweist, entsprechend eine erste und eine zweite Protuberanz (6a, 6b), die voneinander beabstandet entlang dem Inhalationskanal (3) und auf gegenüberliegenden Seiten in Bezug auf eine Mittelebene des Inhalationskanals selbst angeordnet sind.

3. Inhalator (1) nach Anspruch 1 oder 2, **dadurch gekennzeichnet, dass** der zweite Körper (9) einen Sitz (12) zur Einhausung von mindestens einem Abschnitt des ersten Körpers (8) umfasst, wobei der letztere in Bezug auf den zweiten Körper (9) innerhalb des Sitzes (12) zur Einhausung drehbeweglich ist.

4. Inhalator (1) nach einem oder mehreren der vorhergehenden Ansprüche, **dadurch gekennzeichnet, dass** der Inhalationskanal (3) von dem Durchgangstyp ist und einen ersten Zugang (15), der an der gegenüberliegenden Seite zu der äußeren Öffnung (4) in Bezug auf die mindestens eine Protuberanz (6a, 6b) angeordnet ist, und mindestens einen zweiten Zugang (16) aufweist, der an seiner Seitenfläche (11) definiert ist, wobei die ersten und zweiten Zugänge (15, 16) in dem Abschnitt des ersten Körpers (8) definiert sind, der in den Sitz (12) zur Einhausung einführbar ist und der zweite Zugang (16) geeignet ist, um in der zweiten Position des ersten Körpers (8) den Behälter (5) mit dem Inhalationskanal (3) in Verbindung zu bringen.

5. Inhalator (1) nach Anspruch 4, **dadurch gekennzeichnet, dass** die äußere Öffnung (4) und der erste Zugang (15) entsprechend an den axialen Enden des ersten Körpers (8) angeordnet sind und dadurch, dass der zweite Zugang (16) zwischen der äußeren Öffnung (4) und dem ersten Zugang (15) angeordnet ist.

6. Inhalator (1) nach Anspruch 4 oder 5, **dadurch gekennzeichnet, dass** die Prallfläche (7a, 7b) in Richtung des ersten Zugangs (15) gedreht ist.

7. Inhalator (1) nach einem der vorhergehenden Ansprüche, sofern rückbezogen auf Anspruch 2, **dadurch gekennzeichnet, dass** die erste Protuberanz (6a) stromaufwärts von der zweiten Protuberanz (6b) und an der gegenüberliegenden Seite zu dem zweiten Zugang (16) in Bezug auf die Mittelebene angeordnet ist.

8. Inhalator (1) nach einem der vorhergehenden Ansprüche, **dadurch gekennzeichnet, dass** der zweite Körper (9) eine Lufteinlassöffnung (18) aufweist, die mit dem Inhalationskanal (3) in Verbindung steht.

9. Inhalator (1) nach einem der vorhergehenden Ansprüche, **dadurch gekennzeichnet, dass** er mindestens eine Abdeckung (20) aufweist, die auf eine abnehmbare Weise mit dem zweiten Körper (9) verbunden und zum Verschließen der Einführöffnung (19) geeignet ist.

## Revendications

1. Inhalateur (1) pour préparations pulvérulentes, comprenant un corps principal (2) comprenant :
- au moins un premier corps (8) de forme allongée et définissant un conduit d'inhalation (3) s'étendant selon un axe longitudinal (10) dudit premier corps (8), ledit conduit d'inhalation (3) comprenant au moins une ouverture extérieure (4) accessible à l'usager ;
- au moins un second corps (9) définissant un réservoir (5) apte à contenir au moins une dose de la préparation pulvérulente (P) et à être ouvert afin d'être mis en communication avec ledit conduit d'inhalation (3) ; et
- au moins une protubérance (6a, 6b) faisant saillie de la surface latérale (11) dudit conduit d'inhalation (3) transversalement par rapport audit axe longitudinal (10) ;
dans lequel ledit premier corps (8) est mobile en rotation par rapport audit second corps (9) autour dudit axe longitudinal (10) entre au moins une première position, dans laquelle ledit premier corps (8) ferme ledit réservoir (5) pour l'isoler dudit conduit d'inhalation (3), et au moins une seconde position, dans laquelle ledit premier corps (8) ouvre ledit réservoir (5) le mettant en communication avec ledit conduit d'inhalation (3), et
dans lequel ladite protubérance (6a, 6b) défini au moins une surface d'impact sensiblement concave (7a, 7b) disposée du côté opposé à ladite ouverture extérieure (4), ladite surface d'impact (7a, 7b) étant alors destinée à être interceptée par ladite préparation pulvérulente (P) lors de son passage à travers ledit conduit d'inhalation (3) vers ladite ouverture extérieure (4) ;
l'inhalateur (1) **étant caractérisé en ce que** ledit réservoir (5) comprend une bouche d'introduction (19), permettant l'introduction de ladite préparation pulvérulente (P), accessible de l'extérieur, et que ladite surface d'impact (7a, 7b) est sensiblement en forme de C.

2. Inhalateur (1) selon la revendication 1, **caractérisé en ce qu'**il comprend deux desdites protubérances, respectivement une première et une seconde protubérance (6a, 6b), disposées de façon à être espacées l'une par rapport à l'autre le long dudit conduit d'inhalation (3) et disposées sur des faces opposées par rapport à un plan médian du conduit d'inhalation lui-même.

3. Inhalateur (1) selon la revendication 1 ou 2, **caractérisé en ce que** ledit second corps (9) comprend un siège de logement (12) d'au moins une portion dudit premier corps (8), ce dernier étant mobile en rotation par rapport audit second corps (9) à l'intérieur dudit siège de logement (12).

4. Inhalateur (1) selon l'une ou plusieurs des revendications précédentes, **caractérisé en ce que** ledit conduit d'inhalation (3) est du type traversant et comporte un premier port (15) disposé sur le côté opposé à ladite ouverture extérieure (4) par rapport à ladite au moins une protubérance (6a, 6b) et au moins un second port (16) défini sur sa surface latérale (11), lesdits premier et second ports (15, 16) étant définis dans la portion dudit premier corps (8) insérable dans ledit siège de logement (12) et ledit second port (16) étant apte à mettre en communication ledit réservoir (5) avec ledit conduit d'inhalation (3) dans ladite seconde position du premier corps (8).

5. Inhalateur (1) selon la revendication 4, **caractérisé en ce que** ladite ouverture extérieure (4) et ledit premier port (15) sont disposés en correspondance avec les extrémités axiales dudit premier corps (8) et **caractérisé en ce que** ledit second port (16) est disposé entre ladite ouverture extérieure (4) et ledit premier port (15).

6. Inhalateur (1) selon la revendication 4 ou 5, **caractérisé en ce que** ladite surface d'impact (7a, 7b) est orientée vers ledit premier port (15).

7. Inhalateur (1) selon l'une quelconque des revendications précédentes lorsqu'elle dépend de la revendication 2, **caractérisé en ce que** ladite première protubérance (6a) est agencée en amont de ladite seconde protubérance (6b) et sur la face opposée audit second port (16) par rapport audit plan médian.

8. Inhalateur (1) selon l'une quelconque des revendications précédentes, **caractérisé en ce que** ledit second corps (9) comprend une bouche d'entrée d'air (18) en communication avec ledit conduit d'inhalation (3).

9. Inhalateur (1) selon l'une quelconque des revendications précédentes, **caractérisé en ce qu'**il comprend au moins un couvercle (20) associé de manière amovible audit second corps (9) et apte à obturer ladite bouche d'introduction (19).
